# EUROPÄISCHE PATENTSCHRIFT

(11) **EP 1 797 174 B1**
(45) Veröffentlichungstag und Bekanntmachung des Hinweises auf die Patenterteilung: **14.04.2010**
(21) Anmeldenummer: 05790041.7
(22) Anmeldetag: 07.10.2005
(51) Int. Cl.: C12N 7/02

(54) **VERFAHREN ZUR HERSTELLUNG VON VIRUSMATERIAL**
METHOD FOR PREPARING VIRAL MATERIAL
PROCEDE DE PRODUCTION D'UNE MATIERE VIRALE

(30) Priorität: 09.10.2004 DE 102004049290
(43) Veröffentlichungstag der Anmeldung: 20.06.2007
(73) Patentinhaber: AiCuris GmbH & Co. KG, 42117 Wuppertal (DE)
(72) Erfinder: POHLSCHEIDT, Michael, 53844 Troisdorf (DE); BÖDEKER, Berthold, 42113 Wuppertal (DE); MINUTH, Torsten, 42109 Wuppertal (DE); APELER, Heiner, 42111 Wuppertal (DE); LANGER, Uwe, 42285 Wuppertal (DE); BRABENDER, Katrin, 42349 Wuppertal (DE); OTTO-BRABENDER, Dirk, 42349 Wuppertal (DE); KERPER, Joachim, 42389 Wuppertal (DE); HENZLER, Hans-Jürgen, 42655 Solingen (DE)
(74) Vertreter: Findeisen, Marco
(86) Internationale Anmeldenummer: PCT/EP2005/010810
(87) Internationale Veröffentlichungsnummer: WO 2006/040084

(56) Entgegenhaltungen:
- WO-A-98/33886
- WO-A-99/57297
- CHUNG I S TATICEK R A ET AL: "Production of human alkaline phosphatase, a secreted, glycosylated protein, from a baculovirus expression system and the attachment-dependent cell line Trichoplusia ni BTI-Tn 5B1-4 using a split-flow, air-lift bioreactor" BIOTECHNOLOGY PROGRESS, Bd. 9, Nr. 6, November 1993 (1993-11), Seiten 675-678, XP002318385 ISSN: 8756-7938

## Beschreibung

### Das technische Gebiet

Die Erfindung betrifft ein Verfahren zur Herstellung von Virussuspensionen. Die Erfindung betrifft insbesondere ein Verfahren zur Herstellung von hochtitrigen Virussuspensionen in Zellkulturen. Bevorzugte Verfahren beinhalten eine Volumenerhöhung der Zellkultur vor der Infektion mit Virusmaterial und nachfolgend weitere Schritte oder Volumenvergrößerung auf ein Endvolumen, welches deutlich größer ist, als das maximale Kulturvolumen vor der Infektion.

### Stand der Technik

Im Stand der Technik sind verschiedene Verfahren zur Herstellung von Virusmaterial bekannt. Insbesondere sind Verfahren bekannt, bei denen das Virusmaterial von tierischen Zellkulturen hergestellt wird.

Der Fachmann unterscheidet adhärent wachsende Zelllinien, d.h. Zelllinien welche bevorzugt auf festen Oberflächen wachsen, von Zelllinien, die bevorzugt in Suspension wachsen. Adhärent wachsende Zelllinien werden entweder direkt auf der Oberfläche des verwendeten Kultivierungsgefäßes kultiviert, oder sie wachsen auf festen Partikeln (z.B. auf Microcarriern), welche ihrerseits in einem Nährmedium suspendiert vorliegen können.

Es sind.Verfahren zur Herstellung von Virusmaterial bekannt, die in Suspension wachsende Zelllinien verwenden, als auch solche Verfahren, die adhärent wachsende Zelllinien verwenden.

Von großer Bedeutung bei der Herstellung von Virussuspensionen mit Zellkulturen ist die Medienzusammensetzung. In vielen Fällen müssen fötales Kälberserum (FCS) und Wachstumsfaktoren tierischen oder pflanzlichen Ursprungs zugesetzt werden. Neben Chargenschwankungen und störenden Proteinkomponenten in der Aufarbeitung bedeutet die Verwendung von Seren ein biologisches Sicherheitsrisiko (BSE/ TSE, Mycoplasmen, Prionen etc.). Es sind daher serumfreie, nach Möglichkeit synthetische Medien, zu bevorzugen [MERTEN ET. AL. 1994].

Insbesondere bei der Verwendung von adhärenten Zellen, z.B. bei Microcarrier-Kulturen, ergeben sich neben den typischen technischen Barrieren der Maßstabsvergrößerung, wie z.B. die Aufrechterhaltung einer ausreichenden Sauerstoffversorgung, das Entfernen von CO₂, eine ausreichendende Homogenisierung der Fermenterkultur bei minimaler Scherbeanspruchung, auch und insbesondere Probleme bei der Inokulation des nächst größeren Prozeßmaßstabes [GLACKEN ET. AL. 1990, J.B. GRIFFITHS ET. AL. 1985, AMERSHAM 2001].

Die "direkte Migration" rein adhärent wachsender Zellen von Carrier zu Carrier kann dabei nur durch Manipulation des Prozesses dergestalt erfolgen, dass die Zellen durch die Manipulation zumindest teilweise ihre Adhärenz verlieren. Strategien zur Aufhebung der Adhärenz von adhärent wachsenden Zellen und hierfür verwendbare Enzyme sind dem Fachmann bekannt [E. LINDNER ET. AL. 1987, AMERSHAM 2001, DÜRRSCHMID ET. AL. 2003] und müssen bei der Entwicklung des Prozesses hinsichtlich der Abtrennung oder Inaktivierung der verwendeten Enzyme beachtet werden. In kleineren Maßstäben wurden in den 1970er und 1980er Jahren erfolgreiche Experimente zur direkten Zellmigration von den Behälteroberflächen auf Microcarrier in Rollerflaschen, Petrischalen und T-Flaschen durchgeführt. Erfolgreiche Migration von Carrier zu Carrier adhärent wachsender Zellen ist dem Fachmann lediglich in Festbettreaktoren bekannt, wobei einschränkend bemerkt werden muß, dass es sich hier um Zelllinien handelt, die sowohl in Suspension, als auch adhärent wachsen [AMERSHAM 2001, DÜRRSCHMID ET. AL. 2003].

Eine große Rolle spielt insbesondere die Art der Prozeßführung. In der Literatur sind verschieden Methoden beschrieben, wie beispielsweise Batch- oder Perfusionskulturen. Perfusionskulturen werden dabei zur Entkopplung der Verweilzeit von der spezifischen Wachstumsrate, zur Vermeidung von Inhibitionen oder Limitationen aus dem Kulturmedium zu einer Erhöhung der Produktivität verwendet, wobei diese Kulturen oft über mehrere Monate im "High Density Cell Culture" (HDCC) Betrieb gefahren werden Diese Systeme erfordern jedoch neben aufwendigen peripheren Einrichtungen (Abscheider, Spinfilter, Ultraschall-Zellrückhaltung, etc.) langwierige und komplexe Anfahrperioden [M. REITER ET. AL. 1989, GLACKEN ET. AL. 1990, J.B. GRIFFTTHS ET. AL 1985, AMERSHAM 2001, DÜRRSCHMID ET. AL. 2003A]

Eine weitere Möglichkeit zur ausreichenden Versorgung mit Nährstoffen, bildet die Fütterung der Zellkultur mit hoch konzentrierten Substratlösungen. Insbesondere bei HDCC Betrieb können aus einer Fütterung resultierende Inhibitionen, beispielsweise Ammonium und/oder Laktat, zu geringeren Ausbeuten und Produktivitäten führen. Zur Vermeidung inhibitorischer Konzentrationen empfehlen sich bis dato Perfusions- oder Dialysesysteme.

Im Bereich der Herstellung von Virusmaterial mittels tierischer Zellkultur, in dem komplexe gekoppelte Kinetiken der Zellen und des Virus betrachtet werden müssen, können insbesondere bei der Verwendung von Microcarrier-Zellkulturen Probleme in der Prozeßführung auftreten.

Beispielsweise ist der Nutzen der Propagation eines CPE (engl., *cytopathic effect*) verursachenden Virus durch komplexe Perfusion fraglich, da die Viren die Zellen meist in kurzen Zeiträumen (teilweise weniger als 3 bis 7 Tage nach Infektion) zerstören bzw. lysieren.

In der Literatur sind im Pilot- und Produktionsmaßstab (50 bis 1000 1) Batchprozesse zur Virusvermehrung beschrieben. Die Virusvermehrung erfolgt bei allen beschriebenen Microcarrier-Prozessen mit relativ geringen Zelldichten. Nach Infektion mit dem zu propagierenden Virus verläuft die Infektion bis zur Ernte etwa im späteren Endvolumen des Produktionsmaßstabs [B. MONTAGNON ET. AL. 1984, B. BAIJOT ET. AL. 1987]. In einigen Fällen sind Perfusionskulturen für langsam oder nicht lysierende Viren im Labormaßstab beschrieben. In einem Fall wird ein Medienwechsel auf das Ursprungsvolumen beschrieben. [AMEMHAM 2001]

US 6,455,298-B1 und US 6,656,720-B2 beschreiben ein Verfahren zur Herstellung von Influenza-Virusmaterial mit suspendiert wachsenden Zelllinien. Das offenbarte Verfahren beinhaltet eine erste Kultivierungsphase, in dem das Zellmaterial in Suspensionskultur vermehrt wird, einen Infektionsschritt, und darauffolgend eine zweiten Kultivierungsphase, in der das Virus produziert wird. Während dieser Phase kann die Kultur durch Zugabe von Medium weiter verdünnt werden, oder in der Art einer Perfusionskultur geführt werden. Vorteilhaft an diesem Verfahren ist die Tatsache, dass die Kapazität des Verfahrens nicht durch die begrenzte Ausdehnung der inneren Oberfläche der Kultivierungsgefäße limitiert ist.

Nachteilig ist jedoch, dass in Suspensionskultur nicht so hohe Zelldichten erreicht werden können, wie sie mit Microcarrier-basierten Methoden für die Virusproduktion möglich sind. Weiterhin ist die Abtrennung von Zellmaterial vom Nährmedium bei Suspensionskulturen weitaus aufwendiger als dies bei Microcarrier-basierten Verfahren der Fall ist. Diese Nachteile werden in Verfahren nach der vorliegenden Erfindung umgangen, da hier adhärent wachsende Zelllinien auf Microcarriern zur Herstellung von Virusmaterial verwendet werden.

US 6,726,907 und WO 95/24468 beschreiben Verfahren zur Herstellung von Virusmaterial umfassend eine erste Kultivierungsphase zur Vermehrung des Zellmaterials, einen Infektionsschritt und eine sich daran anschließende zweite Kultivierungsphase, in der das Virusmaterial produziert wird. Während der zweiten Kultivierungsphase findet, im Gegensatz zu den erfindungsgemäßen Verfahren, keine weitere Zugabe von Medium statt, so dass das Kulturvolumen während der zweiten Kultivierungsphase nicht weiter erhöht wird. Daraus resultiert ein relativ geringes Erntevolumen wobei die Kultur außerdem noch einen im Vergleich zum erfindungsgemäßen Verfahren geringeren Virustiter aufweist.

US 5,994,134, US 5,719,051 und US 6,194,210 offenbaren Microcarrier-basierte Verfahren zur Herstellung von Virusmaterial, welche ebenfalls eine erste Kultivierungsphase, einen Infektionsschritt und eine zweite Kultivierungsphase beinhalten. Im Unterschied zu Verfahren nach der vorliegenden Erfindung geht diese zweite Kultivierungsphase nicht mit einer Erhöhung des Kulturvolumens einher, sondern diese zweite Kultivierungsphase wird als Perfusionskultur ausgeführt. Es erfolgt ein kontinuierlicher Zustrom an frischem Medium während ein gleich großer Volumenstrom an Kulturmedium abgezogen wird, so dass das Kulturvolumen dabei konstant bleibt. Vorteilhaft ist bei diesem Verfahren gegenüber dem oben beschriebenen Verfahren mit suspendierten Zelllinien, dass zum einen eine größere Zelldichte erreicht werden kann und zum anderen große Mengen virushaltigen Kulturmediums über einen längeren Zeitraum hinweg geerntet werden können. Dieses Microcarrier-basierte Verfahren zur Herstellung von Virusmaterial weist jedoch den Nachteil auf, dass die dabei gewonnenen virushaltigen Kulturmedien einen im Vergleich zu den erfmdungsgemäßen Verfahren geringeren Virustiter (Viruspartikel pro Volumeneinheit) aufweisen. Dieses erschwert die Isolierung des Virusmaterials und erhöht dadurch die Kosten des Produkts. Weiterhin stellt die Zuführung von frischem Medium und die gleichzeitige Abführung von virushaltiger Kulturbrühe hohe Anforderungen an die Steriltechnik und erhöht die Gefahr von Kontaminationen. Die Verwendung von Verfahren zur Herstellung von Virusmaterial mit einer zweiten Kultivierungsphase im Perfusionsbetrieb ist nicht möglich, wenn der zu produzierende Virus die Lyse der produzierenden Zelle, und damit einen *cytopathic effect* (CPE) verursacht.

Dies gilt auch für das komplexe Verfahren der externen oder internen Dialyse, bei dem Stoffaustausch über semipermeable Membranen mit einer spezifischen Molekülausschlussgrenze. Hierbei muss das verbrauchte Medium von den Zellen getrennt werden, bevor es im Gegenstromverfahren oder Gleichstrom Verfahren gegen frisches Medium über eine extern angebrachte Membran dialysiert wird. Neben dem verblocken der Membran innerhalb des Moduls durch Zelltrümmer, etc. ist vor allem der apparative Aufwand und die Massstabsvergrößerung problematisch.

### Kurze Beschreibung der Erfindung

In Hinblick auf den oben beschriebenen Stand der Technik ist es nun die der vorliegenden Erfindung zugrunde liegende technische Aufgabe, ein Herstellungsverfahren für Virusmaterial bereit zu stellen, mit der in verhältnismäßig kurzer Zeit große Mengen von Virussuspension produziert werden kann, die das Virusmaterial in hoher Konzentration enthält.

Dieses technische Problem wird erfindungsgemäß durch ein Verfahren zur Herstellung von Virusmaterial in einer Microcarrier-Zellkultur, umfassend (a) eine erste Kultivierungsphase, die eine Vergrößerung des Zellkulturvolumens durch Zugabe von Medium und Microcarrier-Material umfasst, wobei ein erstes maximales Zellkulturvolumen erreicht wird; (b) einen Infektionsschritt, der nach besagter ersten Kultivierungsphase erfolgt, und der die Zugabe von infektiösem Virusmaterial zu besagter Microcarrier-Zellkultur umfasst; (c) eine zweite Kultivierungsphase, die nach besagtem Infektionsschritt erfolgt und die eine weitere Vergrößerung des Zellkulturvolumens auf ein zweites maximales Zellkulturvolumen umfasst, wobei während der zweiten Kultivierungsphase Virusmaterial erzeugt wird; und (d) einen Ernteschritt zur Gewinnung des Virusmaterials aus der Microcarrier-Zellkultur, dadurch gekennzeichnet, dass besagtes zweites maximales Kulturvolumen zwei bis sieben mal größer ist als besagtes erstes maximales Kulturvolumen. Besagtes zweites maximales Kulturvolumen ist mindestens zweifach größer als besagtes ersten maximales Kulturvolumen.

Vorteilhaft bei den erfindungsgemäßen Verfahren ist insbesondere die Tatsache, dass der Virustiter der Kulturbrühe durch Zufütterung von Medium nach dem Infektionsschritt um das 10-fache gegenüber dem Batch-Prozess gesteigert werden kann. Besonders vorteilhaft ist die Tatsache, dass diese Erhöhung des Virustiters auch erreicht werden kann, wenn nicht konzentriertes Medium nach dem Infektionsschritt zugefüttert wird, so dass das Kulturvolumen während der zweiten Kultivierungsphase nochmals deutlich erhöht wird. Die absolute Menge an produziertem Virusmaterial kann somit gegenüber dem erfindungsgemäßen Verfahren mit Konzentratzufütterung nochmals beträchtlich erhöht werden.

Weitere Ausführungsformen der Erfindung erschließen sich dem Fachmann durch Studium der weiter unten aufgeführten Beispiele und Erläuterungen.

### Abbildungen

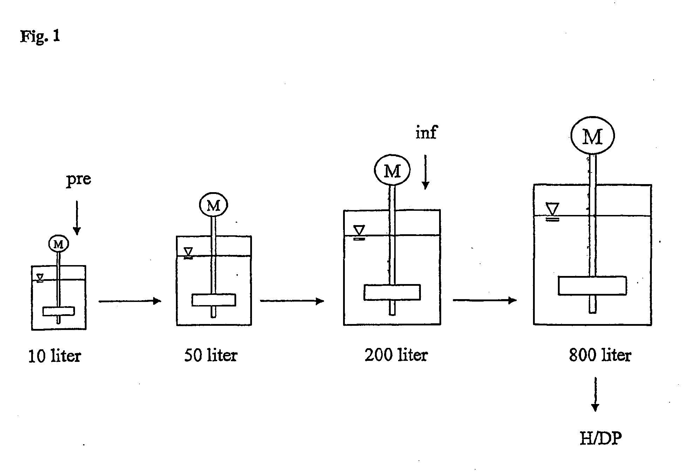

Figur 1: Beispielhafte Darstellung des erfindungsgemäßen Verfahrens mit Volumenänderungen von 10 Liter über 50 Liter und 200 Liter zu 800 Liter. Die Infektion erfolgt im 200 Liter Maßstab. (Abkürzungen: pre, Vorkultur; inf, Infektion; H/DP, Ernte/ Aufarbeitung)

### Detaillierte Beschreibung der Erfindung

Die Erfindung betrifft ein Verfahren zur Herstellung von Virussuspensionen. Erfindungsgemäße Verfahren weisen mindestens 2 Kultivierungsphasen auf. Während der ersten Kultivierungsphase (vor dem Infektionsschritt) wird das Kulturvolumen mehrfach oder kontinuierlich erhöht. Im erfindungsgemäßen Verfahren wird auch nach dem Infektionsschritt das Kulturvolumen schrittweise oder kontinuierlich weiter erhöht, so dass das zu erntende Endvolumen deutlich größer ist, als das maximale Kulturvolumen vor der Infektion.

Die Erfindung betrifft:
1. Ein Verfahren zur Herstellung von Virusmaterial in einer Microcarrier-Zellkultur, umfassend
   (a) eine erste Kultivierungsphase, die eine Vergrößerung des Zellkulturvolumens durch Zugabe von Medium und Microcarrier-Material umfasst, wobei ein erstes maximales Zellkulturvolumen erreicht wird;
   (b) einen Infektionsschritt, der nach besagter ersten Kultivierungsphase erfolgt, und der die Zugabe von infektiösem Virusmaterial zu besagter Microcarrier-Zellkultur umfasst;
   (c) eine zweite Kultivierungsphase, die nach besagtem Infektionsschritt erfolgt und die eine weitere Vergrößerung des Zellkulturvolumens auf ein zweites maximales Zellkulturvolumen umfasst, wobei während der zweiten Kultivierungsphase Virusmaterial erzeugt wird; und
   (d) einen Ernteschritt zur Gewinnung des Virusmaterials aus der Microcarrier-Zellkultur,
   dadurch gekennzeichnet, dass
   besagtes zweites maximales Kulturvolumen zwei bis sieben mal größer ist als besagtes erstes maximales Kulturvolumen.
2. Ein Verfahren nach Punkt 1, wobei besagtes zweites maximales Kulturvolumen drei bis vier mal größer ist als besagtes erstes maximales Kulturvolumen.
3. Ein Verfahren nach einem der Punkte 1 bis 2 bei dem besagte Vergrößerung des Zellkulturvolumens durch Zugabe von nicht konzentriertem Kulturmedium erreicht wird.
4. Ein Verfahren nach einem der Punkte 1 bis 3, bei welchem ein serumfreies Medium verwendet wird.
5. Ein Verfahren nach einem der Punkte 1 bis 4, bei dem im Infektionsschritt eine *multiplicity of infection* (MOI) von 0,001 bis 2 angewendet wird.

Kern der Erfindung ist eine signifikante sequentielle oder kontinuierliche Erhöhung des Produktionsvolumens vorzugsweise mit Medium des gleichen Typs, oder mit Medium eines ähnlichen Typs. Die Steigerung der Effizienz im Vergleich zu klassischen Verfahren ist nachfolgend am Beispiel der Propagation des *Parapoxvirus ovis* beschrieben.

### Beispiele

### Beispiel 1

Zunächst wurde eine bekannte adhärente *Bovine Kidney* Zelllinie in stationären Kulturen (Wannenstapeln, Rollerflaschen) oder in Batch-Microcarrier-Zellkultur kultiviert. Dazu wurde eine Microcarrier-Konzentration von 1 bis 8 g/l, bevorzugt 3 bis 7 g/l, eingesetzt. Die Inokulation des Reaktors erfolgte mit 1 bis 6*10⁵ Zellen/ml. Nachdem die Nährstoffe verbraucht waren, wurde während dieser Zellvermehrungsphase ein Medienwechsel über Sedimentation der Microcarrier durchgeführt. Nach Erreichen der maximalen Zellzahl, 0,2 bis 2*10⁷ Zellen/ml, bevorzugt 0,3 bis 0,7*10⁷ Zellen/ml, wurden im Fall der Kultivierung mit supplementhaltigen Medien zur Reduzierung der Supplementkonzentration, wie beispielsweise FCS, Wachstumshornonen, etc., Waschschritte über mehrfache Sedimentation und Austausch des Überstands mit Medium ohne Supplementierung oder deutlich geringerer Supplementkonzentration durchgeführt. Im Anschluß erfolgte die Infektion mit einer *multiplicity of infection* (MOI) zwischen 0,001 und 2, bevorzugt zwischen 0,005 und 0,1.

Die Infektion fand dabei in einem Kulturvolumen von 10 bis 100 % des Fermentervolumens statt. Die Infektion verlief ohne weitere Manipulation im Batch Modus etwa 3 bis 15 Tage, vorzugsweise 7 bis 11 Tage. Bei Erreichen eines *cytopathic effect* (CPE) der infizierten Zellen zwischen 40 und 100%, bevorzugt 40-90%, erfolgte die Ernte der Kultur.

Die Begasung erfolgt beispielsweise mit blasenfreie und scherarmer Membranbegasung. Der pO₂ wird zwischen 15 und 65%, bevorzugt zwischen 25 und 55%, geregelt.

Der pH Wert wird mit Natriumhydrogencarbonat, Natriumhydroxid und/ oder CO₂-Gas bei pH 6,6 bis pH 7,6, bevorzugt pH 6,9 bis pH 7,5, geregelt. Die Temperatur beträgt zwischen 32°C und 37°C. Die geregelten Parameter in Zellwachstumsphase und Viruspropagationsphase können unterschiedlich sein.

Eine weitere Optimierung der Virusausbeute kann über eine Fütterung mit Mediumkonzentraten bzw. Konzentraten einzelner Substrate während der Viruspropagationsphase erreicht werden. Für verschiedene Systeme ist diese Art der Prozeßführung etabliert und beschrieben. Aber insbesondere bei der Verwendung von adhärenten Zelllinien zur Viruspropagation in Microcarrier-Kulturen gestaltet sich die Bestimmung essentiell notwendiger spezifischer Verbrauchsraten äußerst schwierig, was zum Teil mit den den Experten bekannten Problemen in der Zellzahlbestimmung korreliert. Selbst wenn einzelne Verbrauchsraten für Substrate bekannt sind, stellt sich zusätzlich die Frage nach Inhibitionen der Kultur. In der Literatur sind vor allem Limitationen durch Ammonium oder Laktat beschrieben. Da es keinen allgemein gültigen Schwellenwert gibt, muß dieser spezifisch für das verwendete biologische System bestimmt werden.

Wird eine Limitation/ Inhibition erkannt, muß diese für hohe Produkttiter umgangen werden. In den letzten Jahren wurden daher insbesondere im Bereich der HDCC Perfusions- oder Dialysekulturen eingesetzt, deren Nachteile zur Propagation eines CPE verursachenden Virus bereits diskutiert wurden.

### Beispiel 2

Im folgenden wird das erfindungsgemäße *volume-expanded-fed-batch* (VEF) Verfahren mit den aus dem Stand der Technik bekannten Verfahren verglichen.

Die bereits erwähnte sequentielle Volumenvergrößerung über Verdünnung der Kultur mit frischem Medium nach Infektion (beispielsweise in den nächsten Prozeßmaßstab: 1:2 - 1:7, vorzugsweise 1:3 - 1:5) zeigte dabei erstaunlicher Weise nicht die zu erwartende Erniedrigung des Virustiters, sondern überraschender Weise konnte der Titer trotz Verdünnung im Schnitt 8 bis 13-fach erhöht werden. Dies gelang obwohl das Volumen signifikant um den Faktor 2 bis 7, bevorzugt Faktor 3 bis 4, im Vergleich zum beschriebenen Batch-Prozeß erhöht wurde. Daraus resultiert eine dramatisch verbesserte Virusausbeute.

Mit den beschriebenen und mehrfach durchgeführten Vergleichsverfahren wie beispielsweise die Dialyse, Konzentratfütterungen, Perfusion und/oder durch simple Erniedrigung der Zellzahl, konnten diese Ausbeuten nicht erreicht oder erhöht werden (Tabelle 1).

**Tabelle 1: Gegenüberstellung der bekannten Verfahren mit dem Volume-Expanded-Fed Batch Verfahren beispielhaft beginnend im 3,5Liter Reaktor. Exemplarisch sind Läufe mit vergleichbarer Laufzeit und vergleichbarer Zellzahl zur Propagation des PPVO mittels einer adhärenten BK Zelllinie dargestellt. Die Kultivierungsbedingungen wurden vorab in der Beschreibung des Batchprozesses erwähnt und gelten auch für die anderen Verfahren. Zur Dialyse wurde ein Modul mit einem Molekulargewichtsausschluß von 20 kD verwendet, wobei die Vorkultur im Perfusionsmodus durchgeführt wurde. Die Dialyse wurde im Gegenstromverfahren durchgeführt.Dargestellt sind relative Werte bezogen auf die Batch Kultur.**

| | Batch | Dialyse | Fed-Batch [mit konz. Medium] | VEF-Batch [mit nicht-konzentriertem Medium] |
|---|---|---|---|---|
| TCID₅₀ [relativ zum Batch] | 1 | 10 | 9 | 10 |
| Endvolumen [relativ zum Batch] | 1 | 1 | 1 | 4 |
| Produktivität [relativ zum Batch] | 1 | 10 | 9 | 40 |

Zusätzlich zu den deutlich erhöhten Virusausbeuten resultierende positive Effekte lassen sich vor allem auch im downstream processing erkennen. Diese drücken sich vor allem in geringeren Zellverunreinigungen, wie Host-Cell-Proteinen, Proteinen und DNA pro Dosiseinheit (von besonderer Wichtigkeit bei der Anwendung von vakzinähnlichen humanen Therapeutika), sowie geringeren Ausbeuteverlusten nach Filtration aus. Im Schnitt wird beispielsweise mit einer 20 µm Filtration ein Verlust von ~ 0,6-1 log TCID₅₀ im BatchModus erzielt. Im Vergleich ist im VEF-Batch lediglich ein Verlust von 0,1 -0,4 log TCID₅₀ zu verzeichnen.

Die Vergrößerung des Volumens hat gleichfalls positive Einflüsse auf die bereits beschriebenen technischen Barrieren der Maßstabsvergrößerung. Im Fall der Sauerstoffversorgung, beispielsweise durch das scherarme und Schaumbildung vermeidende Verfahren der Membranbegasung, kann durch eine sukzessive Volumenvergrößerung über Verdünnung eine signifikante Steigerung des Maßstabes erreicht werden (resultierend aus physikalischen Parametern der Reaktoren), da durch die Verringerung der Zellzahl durch Verdünnung und Lyse durch den Virus weniger Sauerstoff in das System eingetragen werden muß. Zum besseren Verständnis ist exemplarisch das Verfahren in einem produktionsrelevanten Maßstabes in Abbildung 1 gezeigt. Die dargestellten Schritte, insbesondere bezüglich der Häufigkeit und Effizienz der direkten Migration beim Übertrag der BK Zelle in den nächsten Fermentationsmaßstab wurden in Experimenten bestätigt. Dabei wurden keine Auswirkungen auf die Produktivität festgestellt.

Die beispielhafte Darstellung beginnt mit der Inokulation des 10 Liter Reaktors. Die Prozessführung erfolgt wie zuvor für den Batchmodus beschrieben. Nach Erreichen der Konfluenz erfolgt eine direkte 1:5 Verdünnung in den 50 Liter Maßstab mit frischen Microcarriern im selben oder vergleichbaren Verhältnis zum Medium wie im 10 Liter Maßstab. Nach abermaligem Erreichen der Konfluenz wird mit dem selben Verfahren der 200 Liter Reaktor inokuliert. Eine kurzzeitige Sedimentation mit oder ohne internierendem Rühren kann von Vorteil sein.

Wenn während der Wachstumsphase serumhaltiges/ proteinhaltiges Medium verwendet wurde erfolgen Waschschritte zur Erniedrigung der Supplementkonzentration mit serum- und proteinfreien Medium. Die Infektion erfolgt wie im Batchprozeß mit der beschriebenen MOI.

10 -36 Stunden nach Infektion erfolgt dann der Beginn des VEF-Batch. Dabei handelt es sich um ein sicheres und robustes Verdünnen der Suspension mit frischem Kulturmedium, wobei dies im selben Reaktor oder in größeren Reaktoren erfolgen kann. Hierzu ist nur ein geringer Aufwand notwendig. Die 200 Liter Virus-Zellsuspension wird beispielsweise auf 400 Liter, dann auf 600 Liter und letztlich auf 800 Liter schrittweise in adäquaten Zeitintervallen und/oder kontinuierlich erhöht. Dies führte, wie bereits beschrieben und in Tabelle 1 dargestellt, überraschender Weise nicht zu einer Verschlechterung Virusproduktivüät.

Unterschiede zwischen serumhaltig, proteinhaltig und synthetisch kultivierten Zellen konnten dabei nicht festgestellt werden. Am Beispiel der an serumfreie und synthetische Bedingungen adaptierte *Bovine Kidney* Zelllinien wurden keine Unterschiede bezüglich der Migration von Carrier zu Carrier, Zellzahl und Produktivität festgestellt. Das heißt, das Verfahren kann für serumhaltige, proteinhaltige und synthetische Kulturmedien eingesetzt werden.

**Tabelle 2: Exemplarischer Vergleich des TCID₅₀ der adaptierten BK Zelllinie an serumhaltige, serumfreie und synthetische Bedingungen zur Propagation des PPVO Virus mit verschiedenen Verfahren bezogen auf Normtiter (hier serumhaltig)**

| | Serumhaltig | Proteinhaltig | Synthetisch |
|---|---|---|---|
| Rollerflasche | 1 | 0,9 | 1 |
| BatchKultur | 1 | 1,1 | 1 |
| VEF-Batch | 1 | 0,9 | 1,1 |

Die erfindungsgemäßen Vorteile des beschriebenen "Volume-Expanded Fed-Batch" gegenüber den etablierten und bekannten Verfahren können wie folgt zusammengefaßt werden: (1) Es handelt sich um ein sicheres, robustes und effizientes Verfahren zur Viruspropagation. (2) Es werden höhere Virusausbeuten erreicht..(3) Es ist kein erhöhter apparativer Aufwand erforderlich. (3) Die Prozeßführung ist vergleichsweise einfach. (4) Der Personalaufwand für die Betreuung des Prozesses ist gering. (5) Die Produktqualität wird besonders in Hinblick auf die folgenden Aufarbeitungsschritte verbessert. (6) Es können größere Fermenter genutzt werden. (7) Einfache Skalierbarkeit ist gegeben. (8) Das Verfahren ist adaptierbar an verschiedene serumhaltige, serumfreie, proteinhaltige und synthetische Kulturmedien

### Beispiel 3

Propagation des *PPVO* im 10 Liter Rührkessel mittels Microcarrier-Zellkultur im Batchmodus unter Verwendung eines proteinhaltigen Mediums.

Die an serumfreie, aber proteinhaltige Bedingungen adaptierte *Bovine Kidney* Zelllinie, wurde ausgehend von der Zellbank zunächst in T- Flaschen und dann in Rollerflaschen kultiviert. Die Kultivierung erfolgte bei 37°C und einem pH Wert von 7,2 +/- 0,2 im CO₂ begasten Brutschrank. Die Ernte des Zellmaterials erfolgte durch Trypsinierung.

Die Konzentration der nach Händleranweisung vorbereiteten Cytodex 3 Microcarriern, Amersham, Schweden, betrug 3 g/l. Die Inokulation erfolgte in 10 Liter mit einer Zellzahl von 2 E05 Z/ml. Während der Zellkultievierungsphase wurde bei einer Glukosekonzentration c < 0,5 g/l ein Medienwechsel mittels Sedimentation durchgeführt. Der Reaktor wurde mit Hilfe eines Ankerrührers mit 30 rpm gerührt. Der pO₂ wurde bei 40% +/- 10% geregelt. Der pH Wert betrug 7.2 +/- 0,2.

Nach 10 Tagen wurde eine Zellzahl von 3,1 E06 Z/ml erreicht, wobei sich die Zellen in der stationären Wachstumsphase befanden. Nach drei Waschschritten mit Medium ohne Supplementen erfolgte die Infektion mit *PPVO* (MOI=0,01) im Endvolumen.

Während der anschließenden Viruspropagation wurden keine Manipulationen vorgenommen. Nach 8 Tagen wurde eine CPE von' 90% erreicht. Die Fermentation wurde nach Sedimentation durch 20µm Filtration abgebrochen. In Tabelle 3 ist der TCID₅₀ zum Zeitpunkt der Ernte und nach Ernte dargestellt.

**Tabelle 3: TCID₅₀ in exemplarisch beschriebener Batch Fermentation.**

| | **Vor Ernte** | **Nach Ernte** | **Volumen** |
|---|---|---|---|
| log ₁₀(TCID₅₀) [log ₁₀ (1/ml)] | 6,7 +/- 0,3 | 6,0 +/- 0,3 | ~ 10 L |

### Beispiel 4:

Propagation des *PPVO* im 3,5 Liter Rührkessel mittels Microcarrier-Zellkultur im Volume-Expanded-Fed Batch (Übertrag in 15 Liter Reaktor) unter Verwendung eines proteinhaltigen Mediums.

Die an serumfreie, aber proteinhaltige Bedingungen adaptierte *Bovine Kidney* Zelllinie, wurde ausgehend von der Zellbank zunächst in T- Flaschen und dann in Rollerflaschen kultiviert. Die Kultivierung erfolgte bei 37°C und einem pH Wert von 7,2 +/- 0,2 im CO₂ begasten Brutschrank. Die Ernte des Zellmaterials erfolgte durch Trypsinierung.

Die Konzentration der nach Händleranweisung vorbereiteten Cytodex 3 Microcarriern, Amersham, Schweden, betrug 5 g/l. Die Inokulation erfolgte in 3,5 Liter mit einer Zellzahl von 3 x 10E5 1/ml. Während der Zellkultivierungsphase wurde bei einer Glukosekonzentration c < 0,5 g/l ein Medienwechsel mittels Sedimentation durchgeführt. Der Reaktor wurde mit Hilfe eines Ankerrührers mit 45 rpm gerührt. Der pO₂ wurde bei 40% +/-10% geregelt. Der pH Wert betrug 7.2 +/- 0.2.

Nach 10 Tagen wurde eine Zellzahl von 7.1 x 10^6 1/ml erreicht, wobei sich die Zellen in der stationären Wachstumsphase befanden. Nach drei Waschschritten mit Medium ohne Supplementen erfolgte die Infektion mit *PPVO* (MOI = 0.01) in 1.7 Liter für 2 h bei n = 14 rpm, bevor auf 3,5 Liter aufgefüllt wurde und die Rührerdrehzahl auf n = 45 rpm erhöht wurde.

16 h nach Infektion wurde die gesamte Kultur in den 15 Liter Reaktor übertragen und 7 Liter aufgefüllt (1:2 Verdünnung). Im 15 Liter Reaktor wurden die gleichen Parameter geregelt

46 h nach Infektion wurde auf 10,5 Liter verdünnt (1:3 Verdünnung bezogen auf 3,5 L). Der CPE betrug bezogen auf die Zellzahl im 3,5 Liter Reaktor und unter Berücksichtigung der Verdünnung etwa 30%.

70 h nach Infektion wurde das Volumen auf 12,5 Liter erhöht, bevor letztlich 94 h nach Infektion auf 13,8 Liter erhöht wurde (1:3,9 Verdünnung).

7 Tage nach Infektion (2,5 Tage nach der letzten Verdünnung) wurde die Fermentation durch Sedimentation und anschließender 20µm Filtration der Kultur abgebrochen (CPE = 93%).

In Tabelle 4 ist der TCID₅₀ zum Zeitpunkt der Ernte und in der Ernte dargestellt.

**Tabelle 4: TCID₅₀ im erfindungsgemäßen VEF-Batch.**

| | Vor Ernte | Nach Ernte | Volumen |
|---|---|---|---|
| log₁₀ (TCID₅₀) [log ₁₀ (1/ml)] | 7,7 +/- 0,3 | 7,6 ⁺/₋ 0,3 | 14 liter |

### Beispiel 5:

Propagation des *PPVO* im 3,5 Liter Rührkessel mittels Microcarrier-Zellkultur im Volumen-Expanded-Fed Batch (Übertrag in 15 Liter Reaktor) unter Verwendung eines proteinfreien und serumfreien Mediums.

Die an synthetische Bedingungen adaptierte *Bovine Kidney* Zelllinie, wurde ausgehend von der Zellbank zunächst in T- Flaschen und dann in Rollerflaschen kultiviert. Die Kultivierung erfolgte bei 37°C und einem pH Wert von 7,2 +/- 0,2 im CO₂ begasten Brutschrank. Die Ernte des Zellmaterials erfolgte durch Trypsinierung.

Die Konzentration der nach Händleranweisung vorbereiteten Cytodex 3 Microcarriern, Amersham, Schweden, betrug 5 g/l. Die Inokulation erfolgte in 3,5 Liter mit einer Zellzahl von 3,8 x 10^5 1/ml. Während der Zellkultivierungsphase wurde bei einer Glukosekonzentration c < 0,5 g/l ein Medienwechsel mittels Sedimentation durchgeführt. Der Reaktor wurde mit Hilfe eines Ankerrührers mit 45 rpm gerührt. Der pO₂ wurde bei 40% +/-10% geregelt Der pH Wert betrug 7.2 +/- 0,2.

Nach 13 Tagen wurde eine Zellzahl von 5,6 E06 Z/ml erreicht, wobei sich die Zellen in der stationären Wachstumsphase befanden. Nach drei Waschschritten mit gleichem Medium erfolgte die Infektion mit *PPVO* (MOI=0,01) in 3,5 Liter n=40 rpm.

20 h nach Infektion wurde die gesamte Kultur in den 15 Liter Reaktor übertragen und 7 Liter aufgefüllt (1:2 Verdünnung). Im 15 Liter Reaktor wurden die gleichen Parameter geregelt.

49 h nach Infektion wurde auf 11 Liter verdünnt (1:3 Verdünnung bezogen auf 3,5 L). Der CPE betrug bezogen auf die Zellzahl im 3,5 Liter Reaktor und unter Berücksichtigung der Verdünnung etwa 30%.

69 h nach Infektion wurde das Volumen auf 12,5 Liter erhöht, bevor letztlich 86 h nach Infektion auf 13,Liter erhöht wurde (1:3,9 Verdünnung).

7 Tage nach Infektion wurde die Fermentation durch Sedimentation und anschließender 20µm Filtration der Kultur abgebrochen (CPE = 93%).

In Tabelle 5 ist der TCID₅₀ zum Zeitpunkt der Ernte und nach der Ernte dargestellt.

**Tabelle 5: Darstellung des erreichten TCID₅₀ in exemplarisch beschriebener VEF- Batch Fermentation mit der synthetischen Zelllinie.**

| | Vor Ernte | Nach Ernte | Volumen |
|---|---|---|---|
| log₁₀(TCID₅₀) TCID₅₀ [1/ml] | 7,8 +/- 0,3 | 7,4 ⁺/₋ 0,3 | ~14 L |

### Beispiel 6

Zur Gewinnung hochreiner Viruspräparate wurden Microcarrier-freie Virusernten eingesetzt. Die Viruspropagation erfolgte z.B. wie in den Beispielen 1 bis 5 beschrieben. Zunächst wurde mit der Virusernte eine schonende Mikrofiltration durchgeführt. Zu diesem Zweck kann z.B ein Kassettenhalter der Fa. Sartorius (Deutschland) mit einer Membrankassette der Fa. Sartorius (Deutschland) eingesetzt werden. Alternativ können auch Hohlfasermodule der Fa. Minntech (USA) oder der Fa. Pall (USA) verwendet werden. Für die Mikrofiltration werden bevorzugt Membranen oder Hohlfasern mit einer Porengröße von 0.1 µm verwendet. Die Mikrofiltrationsstufe dient einer 5- bis 20-fachen Volumenreduktion, der pH-Konditionierung (bevorzugt pH 7.5 bis 9.0) und der Abreicherung von niedermolekularen Fermentationsbegleitstoffen. Das auf diese Art gewonnene Viruskonzentrat wurde mit Ethylenimin bei pH 8.6 chemisch inaktiviert. Für die Virusinaktivierung wurde eine Ethyleniminkonzentration von 3 bis 20 mM eingesetzt. Die Inaktivierung wurde zweistufig durchgeführt. Das Reaktionsgemisch wurde zunächst für 3 bis 6 h bei 37°C unter pH-Kontrolle inkubiert und anschließend wurde die Virusinaktivierung in einem weiteren Reaktionsgefäß bei 37°C über Nacht abgeschlossen. Durch Zugabe eines 1.5 bis 3.0 molaren Überschusses von Natriumtbiosulfat wurde die inaktivierte Virussuspension neutralisiert. Im Anschluß an die Neutralisation erfolgte eine niedertourige Zentrifugation bei 4000 bis 8000 g für 2 bis 4 h. Dieser erste Reinigungsschritt diente der Abtrennung der Viruspartikel von der neutralisierten Inaktivierungslösung. Die inaktivierten Viruspartikel können nach dieser ersten Reinigungsstufe bei 2-8°C oder bei <-65°C bis zur Weiterverarbeitung zwischengelagert werden. Die zweite Reinigungsstufe kann z.B. durch niedertourige Zentrifugation unter Verwendung eines 20% Saccharosekissens erfolgen. Alternativ können aber auch Membranadsorber der Fa. Sartorius (Deutschland) oder der Fa. Pall (USA) eingesetzt werden. Die zweite Zentrifugationsstufe würde bei 4000 bis 8000 g über Nacht durchgeführt. Die Bilanzierung des Reinigungsverfahrens erfolgte mittels asymmetrischer Fluß-Feld-Fluß-Fraktionierung (AF4-Analyse) und Refraktometrie sowie quantifizierender Elektronenmikroskopie. In Tabelle 6 und 7 sind typische Ausbeuteverläufe dargestellt.

**Tabelle 6: Bilanzierung des vorstehend beschriebenen Reinigungsverfahrens mittels asymmetrischer Fluß-Feld-Fluß-Fraktionierung (AF4-Analyse) und Refraktometrie.**

| **Versuchs-Nr.** | **Prozeßstufe** | **Probe** | **Gesamtpartikel** | **Kumulative Ausbeute [%]** |
|---|---|---|---|---|
| 508623 | 3.0/0.8 µm | Start | 5,9E13 | 100 |
| | Partikelfiltration | Filtrat | 2,5E13 | 42 |
| | Zentrifugationsstufe | Start | 2,5E13 | |
| | 1 | Sediment | 5,5E12 | 9 |
| | Zentrifugationsstufe | Start | 5,5E12 | |
| | 2 | Sediment | 3,6E12 | 6 |
| 508624 | 3.0/0.8 µm | Start | 6,6E13 | 100 |
| | Partikelfiltration | Filtrat | 2,3E13 | 35 |
| | Zentrifugationsstufe | Feed | 2,3E13 | |
| | 1 | Sediment | 5,9E12 | 9 |
| | Zentrifugationsstufe | Feed | 5,9E12 | |
| | 2 | Sediment | 3,6E12 | 5 |
| 508627 | 3.0/0.8 µm | Start | 3,6E13 | 100 |
| | Partikelfiltration | Filtrat | 1,5E13 | 43 |
| | Zentrifugationsstufe | Feed | 1,5E13 | |
| | 1 | Sediment | 3,1E12 | 8 |
| | Zentrifugationsstufe | Feed | 3,1E12 | |
| | 2 | Sediment | 2,0E12 | 5 |
| | | | *Mittlere Ausbeute* | *5* |

**Tabelle 7: Bilanzierung des vorstehend beschriebenen Reinigungsverfahrens mittels quantifizierender Elektronenmikroskopie.**

| **Versuchs-Nr.** | **Prozeßstufe** | **Probe** | **Gesamtpartikel** | **Kumulative Ausbeute [%]** |
|---|---|---|---|---|
| 508623 | 3.0/0.8 µm | Start | 1,5E13 | 100 |
| | Partikelfiltration | Filtrat | n.d. | - |
| | Zentrifugationsstufe | Feed | n.d. | - |
| | 1 | Sediment | 4,4E12 | 30 |
| | Zentrifugationsstufe | Feed | 4,5E12 | - |
| | 2 | Sediment | 4,2E12 | 29 |
| 508624 | 3.0/0.8 µm | Start | 2,4E13 | 100 |
| | Partikelfiltration | Filtrat | n.d. | - |
| | Zentrifugationsstufe | Feed | n.d. | - |
| | 1 | Sediment | 4,6E12 | 19 |
| | Zentrifugationsstufe | Feed | 4,6E12 | |
| | 2 | Sediment | 3,2E12 | 13 |
| 508627 | 3.0/0.8 µm | Start | 7,3E12 | 100 |
| | Partikelfiltration | Filtrat | n.d. | - |
| | Zentrifugationsstufe | Feed | n.d. | - |
| | 1 | Sediment | 2,3E12 | 31 |
| | Zentrifugationsstufe | Feed | 2,3E12 | - |
| | 2 | Sediment | 3,5E12 | 48 |
| | | | *Mittlere Ausbeute* | *13 - 48* |

Der Wirtszellproteingehalt wurde auf ausgewählten Prozeßstufen mit einem spezifischen Wirtszellproteinassay ermittelt und zur Bestimmung von Abreicherungsfaktoren verwendet. In Tabelle 8 sind typische Abreicherungsergebnisse dargestellt.

**Tabelle 8: Abreicherung von Wirtszellproteinen im Verlauf der Virusreinigung.**

| **Versuchs-Nr.** | **Prozeßstufe** | **Probe** | **HCP Gehalt [µg/1E10 VPs]** | **Kumulative HCP-Abreicherung** |
|---|---|---|---|---|
| 508623 | 3.0/0.8 µm | Prefiltration | 58 | - |
| | Partikelfiltration | Post-filtration | n.d. | - |
| | Zentrifugationsstufe | Feed | n.d. | - |
| | 1 | Sediment | 3,5 | 17 |
| | Zentrifugationsstufe | Feed | 3,5 | - |
| | 2 | Sediment | 3 | 19 |
| 508624 | 3.0/0.8 µm | Prefiltration | 80 | - |
| | Partikelfiltration | Post-filtration | n.d. | - |
| | Zentrifugationsstufe | Feed | n.d. | - |
| | 1 | Sediment | 4,8 | 17 |
| | Zentrifugationsstufe | Feed | 4,8 | |
| | 2 | Sediment | 5 | 16 |
| 508627 | 3.0/0.8 µm | Prefiltration | 187 | - |
| | Partikelfiltration | Post-filtration | n.d. | - |
| | Zentrifugationsstufe | Feed | n.d. | - |
| | 1 | Sediment | 14 | 13 |
| | Zentrifugationsstufe | Feed | 14 | - |
| | 2 | Sediment | 14 | 13 |
| | | | *Mittlere Abreicherung* | *13 -19* |

Die Prüfung auf mikrobielle Reinheit erfolgte mit den üblichen Standardverfahren. Es konnte gezeigt werden, dass das vorstehend beschriebene Reinigungsverfahren unter aseptischen Bedingungen durchgeführt werden kann.

**Tabelle 9: Bewertung der mikrobiellen Reinheit.**

| **Versuchs-Nr.** | **Prozeßstufe** | **Probe** | **Bioburden** [Counts/ml] |
|---|---|---|---|
| 508623 | 3.0/0.8 µm | Start | 0/0 |
| | Partikelfiltration | Filtrat | 0/0 |
| | Zentrifugationsstufe | Feed | - |
| | 1 | Sediment | 0 / 0 |
| | Zentrifugationsstufe | Feed | - |
| | 2 | Sediment | 0/0 |
| 508624 (02KUR02) | 3.0/0.8 µm | Start | 0/0 |
| | Partikelfiltration | Filtrat | 0/0 |
| | Zentrifugationsstufe | Feed | - |
| | 1 | Sediment | 0 / 0 |
| | Zentrifugationsstufe | Feed | - |
| | 2 | Sediment | 0/0 |
| 508627 (02KUR05) | 3.0/0.8 µm | Start | 0/0 |
| | Partikelfiltration | Filtrat | 0/0 |
| | Zentrifugationsstufe | Feed | - |
| | 1 | Sediment | 1 / 0 |
| | Zentrifugationsstufe | Feed | - |
| | 2 | Sediment | 0 / 0 |

Im Anschluß an die zweite Reinigungsstufe wurde die hochreine Viruspräparation unter Verwendung von Mikrofiltration formuliert. Für diese Formulierungsstufe können Membranen der Fa. Sartorius (Deutschland) oder der Fa. Pall (USA) sowie Hohlfasern der Fa. Minntech (USA) oder Amersham Biosciences (USA) eingesetzt werden. Die bevorzugte Porengröße liegt bei 0.1 µm. Zweck dieser Formulierungsstufe ist es, die Virussuspension zu konditionieren hinsichtlich pH-Wert, Osmolarität und Partikelgehalt. Nach Zugabe eines geeigneten Stabilisators (1-5 % Polygeline) kann das so hergestellte Viruspräparat zwecks Langzeitlagerung lyophilisiert werden. Vor seiner Verwendung als Arzneimittel muß das Lyophilisat entsprechend dem Ausgangsvolumen mit sterilem, pyrogenfreiem WO (water for injection) versetzt werden. Die mittels der vorstehend beschriebenen Vorgehensweise hergestellte Viruszubereitung eignet sich für parenterale Applikationen.

In der Tabelle 10 sind typische Ergebnisse, der Charakterisierung des formulierten Viruspräparates vor Gefriertrocknung zusammengestellt.

**Tabelle 10: Analyse der formulierten Virussuspension vor Gefriertrocknung.**

| **Parameter** | **508623** | **508624** | **508627** |
|---|---|---|---|
| Partikelgehalt mittels AF4/RI [VPs/ml] | 1,8E10 | 2,0E10 | 1,0E10 |
| Partikelgehalt mittels quantifizierender Elektronenmikroskopie [VPs/ml] | 4,15E10 | 3,58E10 | 2,5E10 |
| Biologische Aktivität (transgene HBV-Maus) | | entspricht | |
| Bioburden [Counts/ml] | 0 | 0 | 0 |
| Endotoxingehalt [EU/ml]* | 6 | 6 | 2,4 |
| Wirtszellproteingehalt [µg/ml] | 4,7 - 6,4 | 8,6 - 9,3 | 9,9 -13,3 |
| Nukleinsäuregehalt [ng/ml] | 3,7 | 2,9 | 1,6 |

| | | | |
|---|---|---|---|
| *: der verwendete Virusstabilisator hat bereits einen mittleren Endotoxingehalt von 2 bis 8 EU/ml. | | | |

### PATENTE

US 6,455,298
US 6,656,720
US 5,994,134
US 5,719,051
US 6,194,210
US 6,726,907
WO 95/24468

### LITERATUR

AMERSHAM, "Microcarrier Cell Culture- Principals and Methods", Manual Amersham Pharmacia, Sweden, 2001
B. BAIJOT, M. DUCHENE, J. STEPHENNE, "Production of Aujesky Vaccine by the Microcarrier Technology", Devel. Biol. Standard,. 66, 523-530, 1987
M. P. DÜRRSCHMID, K. LANDAUER, G. SIMIC, H. KLUG, T. KEIJZER, F. TRAMPLER, A. OUDSHOORN, M. GRÖSCHL, D. MÜLLER, O. DOLBHOFF-DIER, "Comparison of Fluidized Bed and Ultrasonic Cell Retention Systems for High Cell Density Mammalian Cell Culture", Biotechnol. Prog,. 19, 1045-1048, 2003
M. P. DÜRRSCHMID, K. LANDAUER, G. SIMIC, G. BLÜML, O. DOLBHOFF-DIER, "Scaleable Inoculation Strategies for Microcarrier Based Animal Cell Bioprocesses", Biotechnol. And Bioeng,. 83, No. 6, 2003
M.W. GLACKEN, R.J. FLEISCHAKER, A.J. SINSKEY, "Large Scale Production of Mammalian Cells and Their Products", in Annals New York Academy of Sciences,355-372,1989
J. B. GRIFFITHS, D.R CAMERON, D. LOBBY, "A Comparison of Unit Process Systems for Anchorage Dependet Cells", Devel. Biol. Standard,. 66, 331-338, 1994
E. LINDNER, A.-C. ARVIDSSON, I. WERGELAND, D. BILLIG, "Subpassaging Cells on Microcarriers", Devel. Biol. Standard,. 66, 299-305, 1987
MERTEN, O.W., KIERULFF J.V., CASTINGOLLES, N., PERRIN, P, "Evaluation of the new serum free medium (MSDSS2) for the production of different biologicals: Use of various cell lines", Cytotechnol. 14, 47-59, 1994
B.J. MONTAGNON, B. FANGET, J.C: VINCENT-FALQUET, "Industrial Scale Production of Inactivated Poliovirus Vaccine Prepared by Culture of Vero Cells on Microcarrier" Rev. Of Inf. Diseases,. 6, Supplement 2, 1984
B.J. MONTAGNON, J.C: VINCENT-FALQUET, B. FANGET, "Thousand Litre Scale Microcarrier Culture of Vero Cells for Killed Polio Viruses Vaccine", Devel. Biol. Standard,. 55, 37-42, 1984
M. REITER, F. WEIGANG, W. ERNST, "High Density Microcarrier Culture with a New Device which Allows Oxygenation and Perfusion of Microcarrier Cultures" Cytotechnology,. 3, 39-42, 1990

## Patentansprüche

1. Verfahren zur Herstellung von Virusmaterial in einer Microcarrier-Zellkultur, umfassend
(a) eine erste Kultivierungsphase, die eine Vergrößerung des Zellkulturvolumens durch Zugabe von Medium und Microcarrier-Material umfasst, wobei ein erstes maximales Zellkulturvolumen erreicht wird;
(b) einen Infektionsschritt, der nach besagter erster Kultivierungsphase erfolgt, und der die Zugabe von infektiösem Virusmaterial zu besagter Microcarrier-Zellkultur umfasst;
(c) eine zweite Kultivierungsphase, die nach besagtem Infektionsschritt erfolgt und die eine weitere Vergrößerung des Zellkulturvolumens auf ein zweites maximales Zellkulturvolumen umfasst, wobei während der zweiten Kultivierungsphase Virusmaterial erzeugt wird; und
(d) einen Ernteschritt zur Gewinnung des Virusmaterials aus der Microcarrier-Zellkultur,
**dadurch gekennzeichnet, dass**
besagtes zweites maximales Kulturvolumen zwei bis sieben mal größer ist als besagtes erstes maximales Kulturvolumen.

2. Verfahren nach Anspruch 1, wobei besagtes zweites maximales Kulturvolumen drei bis vier mal größer ist als besagtes erstes maximales Kulturvolumen.

3. Verfahren nach einem der Ansprüche 1 oder 2 bei dem besagte Vergrößerung des Zellkulturvolumens durch Zugabe von nicht konzentrierten Kulturmedium erreicht wird.

4. Verfahren nach einem der Ansprüche 1 bis 3, bei welchem ein serumfreies Medium verwendet wird.

5. Verfahren nach einem der Ansprüche 1 bis 4, bei dem im Infektionsschritt eine *multiplicity of infection* (MOI) von 0,001 bis 2 angewendet wird.

## Claims

1. Method for the production of virus material in a microcarrier cell culture, comprising
(a) a first cultivation phase comprising an increase of the cell culture volume by the addition of medium and microcarrier material, thereby obtaining a first maximum cell culture volume;
(b) an infection step occurring after said first cultivation phase and comprising the addition of infectious virus material to said microcarrier cell culture;
(c) a second cultivation phase occurring after said infection step, and comprising a further increase of the cell culture volume to a second maximum cell culture volume, wherein during the second cultivation phase virus material is generated; and
(d) a harvest step to recover the virus material from the microcarrier cell culture,
**characterized in that**
said second maximum culture volume is two to seven times larger than said first maximum culture volume.

2. Method of claim 1, wherein said second maximum culture volume is three to four times larger than said first maximum culture volume.

3. Method of any of claims 1 or 2, wherein said increase of the cell culture volume is achieved by the addition of non-concentrated culture medium.

4. Method of any of claims 1 to 3, wherein serum-free medium is used.

5. Method of any of claims 1 to 4, wherein in the infection step a *multiplicity of infection* (MOI) of 0.001 to 2 is applied.

## Revendications

1. Procédé de production de matériau viral dans une culture cellulaire à micro-véhicule comprenant
(a) une première phase de culture qui comprend un accroissement du volume de culture cellulaire par l'addition de milieu et de matériau de micro-véhicule, un premier volume maximal de culture cellulaire étant obtenu ;
(b) une étape d'infection qui a lieu après ladite première phase de culture et qui comprend l'addition de matériau viral infectieux à ladite culture cellulaire à micro-véhicule ;
(c) une deuxième phase de culture qui s'effectue après ladite étape d'infection et qui comprend un accroissement supplémentaire du volume de culture cellulaire à un deuxième volume de culture cellulaire maximal, le matériau viral étant produit pendant la deuxième phase de culture ; et
(d) une étape de récolte pour recueillir le matériau viral de la culture cellulaire à micro-véhicule,
**caractérisé en ce que**
ledit deuxième volume de culture maximal est deux à sept fois plus grand que ledit premier volume de culture maximal.

2. Procédé selon la revendication 1, dans lequel ledit deuxième volume de culture maximal est deux à quatre fois plus grand que ledit premier volume de culture maximal.

3. Procédé selon l'une des revendications 1 ou 2, dans lequel ledit accroissement du volume de culture cellulaire est obtenu par addition de milieu de culture non concentré.

4. Procédé selon l'une des revendications 1 à 3, dans lequel on utilise un milieu sans sérum.

5. Procédé selon l'une des revendications 1 à 4, dans lequel à l'étape d'infection, on utilise une MOI (multiplicity of infection) de 0,001 à 2.
